# EUROPÄISCHE PATENTSCHRIFT

(11) **EP 2 043 552 B1**
(45) Veröffentlichungstag und Bekanntmachung des Hinweises auf die Patenterteilung: **25.05.2016**
(21) Anmeldenummer: 07764894.7
(22) Anmeldetag: 27.06.2007
(51) Int. Cl.: A61F 2/06, A61L 31/02, C23C 14/00, B81C 1/00

(54) **VERFAHREN ZUR HERSTELLUNG STRUKTURIERTER SCHICHTEN AUS TITAN UND NICKEL**
A METHOD FOR THE PRODUCTION OF STRUCTURED LAYERS OF TITANIUM AND NICKEL
PROCÉDÉ DE FABRICATION DE COUCHES STRUCTURÉES EN TITANE ET NICKEL

(30) Priorität: 27.06.2006 DE 102006029831
(43) Veröffentlichungstag der Anmeldung: 08.04.2009
(73) Patentinhaber: Acandis GmbH & Co. KG, 76327 Pfinztal (DE)
(72) Erfinder: SCHMUTZ, Clemens, 50996 Köln (DE); QUANDT, Eckhard, 24226 Heikendorf (DE); ZAMPONI, Christiane, 24114 Kiel (DE)
(74) Vertreter: Bohnenberger, Johannes
(86) Internationale Anmeldenummer: PCT/EP2007/005702
(87) Internationale Veröffentlichungsnummer: WO 2008/000467

(56) Entgegenhaltungen:
- WO-A-00/04204
- WO-A-01/87371
- WO-A-2004/008504
- US-B2- 6 791 233
- BUCHAILLOT L ET AL: "Thin film of titanium/nickel shape memory alloy for multi-degree of freedom microactuators" SEISAN KENKYU, TOKYO DAIGAKU SEISANGIJUTSU KENKYUJO, TOKYO, JP, Bd. 51, Nr. 8, 1999, Seiten 22-23, XP009087245 ISSN: 0037-105X
- RUMPF H ET AL: "Near net-shape fabrication of superelastic NiTi devices by sputtering and photoetching" MATERIALS TRANSACTIONS. JIM, SENDAI, JP, Bd. 47, Nr. 3, März 2006 (2006-03), Seiten 523-526, XP009089814 ISSN: 0916-1821
- WALKER J A ET AL: "THIN-FILM PROCESSING OF TINI SHAPE MEMORY ALLOY" SENSORS AND ACTUATORS A, ELSEVIER SEQUOIA S.A., LAUSANNE, CH, Bd. A21, Nr. 1 / 3, 1. Februar 1990 (1990-02-01), Seiten 243-246, XP000149585 ISSN: 0924-4247

## Beschreibung

Die vorliegende Erfindung betrifft ein Verfahren zur Herstellung einer strukturierten Metallschicht aus einer Titan und Nickel umfassenden Legierung nach dem Oberbegriff des Anspruchs 1. Derartig hergestellte strukturierte Metallschichten bzw. Metallfolien können insbesondere als biokompatibles Implantat, beispielsweise als Emboliefilter oder als Bänder bzw. allgemein als Verbindungsglieder zwischen den Knochen des menschlichen Skeletts verwendet werden. Bei geeigneter Formgebung können derartige Schichten auch als Stents in Blutgefäßen eingesetzt werden.

Die zur Herstellung der Metallschicht verwendete Legierung mit Titan und Nickel hat vorteilhafterweise ein superelastisches Verhalten und/oder Formgedächtniseigenschaften. Werkstoffe mit Formgedächtniseigenschaften (FG-Werkstoffe) zeichnen sich insbesondere dadurch aus, dass sie in einer Tieftemperaturphase mit Martensit-Gefüge verformt werden können und sich nach einer anschließenden Erwärmung in einer Hochtemperaturphase mit Austenit-Gefüge an diese aufgeprägte Form erinnern und diese wieder annehmen. Eine häufig genutzte Eigenschaft derartiger Werkstoffe ist das superelastische Verhalten. Innerhalb eines bestimmten Temperaturintervalls oberhalb einer charakteristischen Vorspannung, die einige hundert MPa betragen kann, tritt ein Plateau in der Spannungs-Dehnungs-Kurve auf. In diesem Dehnungsbereich wandelt sich der Austenit in Martensit um. Der spannungsinduzierte Martensit kann sich entsprechend der angelegten Spannung entzwillingen und ermöglicht damit innerhalb des Plateaus eine Deformation des Materials bei konstanter Gegenkraft. Dabei können Dehnungen bis ca. 8% über die Phasentransformation in den spannungsinduzierten Martensit aufgebracht werden, ohne dass plastische Verformung auftritt. Bei Entlastung des Martensites wandelt sich dieser wieder mit einer Hysterese bzgl. der Plateauspannung in den Ausgangszustand des Austenits um.

Auf Grund ihrer guten Biokompatibilität werden Werkstoffe aus Nickel-Titan-Legierungen (NiTi) häufig in der Medizintechnik eingesetzt. Bei medizinischen Werkzeugen wie Kathetern, die beispielsweise zur Stent Positionierung verwendet werden und die während ihres Einsatzes im Körper starken Verformungen ausgesetzt werden, sind die superelastischen Eigenschaften der Nickel-Titan-Legierungen von Vorteil. Gewebespreizer mit superelastischen Eigenschaften haben den Vorteil, dass sie das Gewebe weniger stark schädigen als Spreizer aus anderen Materialien. Zusätzlich lässt sich der Formgedächtniseffekt bei Implantaten wie Stents oder Emboliefiltern ausnutzen. Hierbei werden die Implantate bei Zimmertemperatur im martensitischen Zustand verformt. Anschließend werden die verformten Implantate in den Körper eingesetzt, wo bei Körpertemperatur die Hochtemperaturphase Austenit stabil ist. Dabei wandelt sich das Implantat um und erinnert sich an seine ursprüngliche Form. Die zusammengefalteten Stents und Emboliefilter können sich so selbständig entfalten.

Grundsätzlich kann der Anteil von Nickel in der für die Herstellung der Metallschicht verwendeten Legierung innerhalb großer Grenzen je nach Anwendungsfall zwischen 2 und 98 Atom-% variiert werden. Vorzugsweise wird jedoch vorgeschlagen, dass der Nickel-Gehalt in der Legierung zwischen 45 und 60 Atom-% liegt.

Herkömmlicherweise handelt es sich bei Implantaten um ein Kompaktmaterial, das mit konventionellen Fertigungstechniken hergestellt wird. Es hat sich jedoch herausgestellt, dass poröse Formen des Kompaktmaterials bei medizinischen Implantaten von Vorteil sind, da Zellen, insbesondere Stammzellen oder Knochenzellen, dabei in die Poren hineinwachsen können, so dass eine bessere Einbettung des Implantats in den Knochen bzw. in das neu zu bildende Gewebe gewährleistet ist. So handelt es sich auch bei Stents und Emboliefiltern um netzartige strukturierte Formen. Typischerweise erfolgt die Formgebung der Stents durch eine Kombination aus Tiefbohren und Laserschneiden aus Vollmaterial.

Darüber hinaus ist die Herstellung von dünnen Formgedächtnis-Schichten mit superelastischem Verhalten durch physikalische Abscheidemethoden, insbesondere durch Sputtern bekannt. Gesputterte Schichten aus Nickel-Titan-Legierungen ermöglichen dabei neben dem Vorteil eines geringeren Materialaufwands vor allem die Herstellung kleiner dimensionierter Strukturen.

Zur Einbringung einer Struktur in eine als geschlossene Fläche gesputterte Schicht einer Nickel-Titan-Legierung ist es bekannt, die Nickel-Titan-Schicht einem Ätzprozess zu unterziehen, wobei insbesondere auch Verfahren der Photolithographie zum Einsatz kommen können. Die lithographische Strukturierung von Nickel-Titan-Legierungen erfolgt durch einen Lithographieschritt, in dem die gewünschte Strukturform mittels einer Maskierung auf einen Lack aufgebracht wird. Der lichtempfindliche Lack wird anschließend belichtet und entwickelt. Der hierbei stehen gebliebene Lack schützt, während der weiteren Ätzschritte die Bereiche der Struktur, die erhalten werden sollen.

Nass-chemisch lassen sich Nickel-Titan-Legierungen mittels eines Flusssäuregemischs (HF) ätzen. Der nass-chemische Prozess ist isotrop, so dass hierbei stets auch eine Unterätzung erfolgt und die Kantenstruktur negativ beeinflusst wird. Ferner führt die Unterätzung, deren Grad bei dickeren Schichten zunimmt, auch dazu, dass die Strukturauflösung zerstört wird bzw. die Gitterstegbreiten verloren gehen. Ein weiterer Nachteil besteht in der zurückbleibenden geätzten Oberfläche, die deutliche Ätzspuren enthält, so dass gegebenenfalls eine weitere Oberflächenbehandlung in Form einer Nachpolierung notwendig wird. Außerdem kann während des chemischen Ätzens Wasserstoff in die Nickel-Titan-Legierungen eingebracht werden, was die Formgedächtniseigenschaften bzw. die superelastischen Eigenschaften beeinträchtigen kann.

Ein weiteres Ätzverfahren ist das Trockenätzen, bei dem das zu entfernende Material der Nickel-Titan-Legierung beispielsweise durch einen Argonionenstrahl abgetragen bzw. "abgesputtert" wird. Dieses Verfahren ist anisotrop, so dass eine Unterätzung nicht erfolgt und die Kantenstrukturen sehr sauber hergestellt werden können. Die Einbringung von Wasserstoff wird hiermit ebenfalls vermieden. Ein Nachteil dieses Verfahrens ist jedoch die geringe Ätz-bzw. Abtragrate von wenigen Zehntel nm/s, was einen erheblichen Zeitaufwand bedeutet. Bei Schichtdicken von größer als 2 µm kommt noch das Problem der Redeposition hinzu, bei der sich das schon abgetragene Material erneut auf der Oberfläche festsetzt.

Neben diesen Verfahren, bei denen die Struktur erst nach dem erfolgten Abscheiden in die der Metallschicht eingebracht wird, ist es auch bekannt, eine Struktur in das Trägermaterial einzubringen, auf welches die Nickel-Titan-Legierungs-Schicht anschließend abgeschieden wird und somit unmittelbar in der gewünschten Struktur vorliegt.

So wird bei einem Verfahren der eingangs genannten Art, das aus der WO 00/04204 bzw. aus der korrespondierenden DE 699 20 712 T2 bekannt ist, zunächst ein Opferschichtverbund bereitgestellt, der mindestens zwei aufeinander aufgebrachte Opferschichten umfasst. Eine dieser beiden Opferschichten wird zur Aufbringung einer Struktur einem nasschemischen Ätzprozess unterzogen, wobei es zu einer Unterätzung dieser Opferschicht kommt. Danach wird eine Metallschicht der besagten Legierung mittelbar oder unmittelbar auf den bereits strukturierten Opferschichtverbund aufgebracht.

Bei diesem bekannten Verfahren kommt es aufgrund des isotropen Ätzprozesses zu den oben genannten Nachteilen von Ätzspuren in der Oberfläche der nass-chemisch geätzten Opferschicht. Zur Erreichung einer hohen Bruchfestigkeit einer gesputterten Nickel-Titan-Legierungs-Schicht ist jedoch eine besonders glatte Oberfläche des Substrats bzw. einer zur Abscheidung genutzten Opferschicht von entscheidender Bedeutung. Werden während der Schichtherstellung Risskeime in Form von Kerben oder Poren erzeugt, dann tritt im Zugversuch ein Werkstoffversagen bei weit geringeren Spannungen als der theoretischen Bruchfestigkeit auf. Im Werkstoff werden dann lokale Spannungsspitzen erreicht, die die Bruchfestigkeitsgrenze übersteigen. Solche Spannungsspitzen kommen an Kerben, wie sie Poren im Inneren und Kratzer an der Oberfläche darstellen, durch eine Spannungskonzentration zustande.

Aufgabe der vorliegenden Erfindung ist es, ein einfach durchzuführendes Verfahren der eingangs genannten Art zu schaffen, mit dem strukturierte Nickel-Titan-Schichten mit einer hohen Bruchfestigkeit besonders schnell und kostengünstig hergestellt werden können.

Diese Aufgabe wird erfindungsgemäß durch ein Verfahren nach Anspruch 1 gelöst. Vorteilhafte Ausgestaltungen und Weiterbildungen der Erfindung ergeben sich aus den abhängigen Ansprüchen.

Wesentlich bei der erfindungsgemäßen Lösung ist es, dass die von der abzuscheidenden Metallschicht weiter entfernt liegende erste Opferschicht dem nass-chemischen Ätzprozess unterzogen wird, und dass die der abzuscheidenden Metallschicht zugewandte zweite Opferschicht vor dem nass-chemischen Ätzen der ersten Opferschicht einem Trocken-Ätzprozess unterzogen wird, wobei sie mit einer der gewünschten Struktur der Metallschicht entsprechenden Struktur versehen wird. Die auf die erste Opferschicht aufgebrachte zweite Opferschicht ist dabei vorteilhafterweise chemisch selektiv von der ersten Opferschicht entfernbar.

Der Hauptvorteil liegt dabei darin, dass auf diese Weise aufgrund einer besonders glatten Oberfläche der mit der abgeschiedenen Metallschicht in Kontakt kommenden Opferschicht eine hochfeste Metallschicht erreicht wird, und dass trotzdem die für die Abscheidung einer strukturierten Metallschicht in der Opferschicht benötigten Freiräume in der erforderlichen Größe in sehr kurzer Zeit erzielt werden können.

Durch die erfindungsgemäße Kombination der beiden oben genannten Ätzmethoden und deren gezielte Anwendung auf zwei Opferschichten gelingt es, nur die jeweiligen Vorteile zu nutzen, die vor allem in der Schnelligkeit eines nass-chemischen Prozesses und in der Kantengenauigkeit eines anisotropen trocken-chemischen Ätzprozesses (IBE) liegen.

So wird ein Verfahren geschaffen, mit dem strukturierte Metallschichten bzw. strukturierte Metallfolien aus Nickel-Titan-Legierungen besonders einfach, schnell und kostengünstig hergestellt werden können.

Besonders vorteilhaft ist es, wenn die Metallschicht unmittelbar auf die trockengeätzte zweite Opferschicht aufgebracht wird. Auf diese Weise werden die Vorteile einer besonders glatten Oberfläche der zweiten Opferschicht im Hinblick auf eine optimale Festigkeit der Metallfolie deutlich besser genutzt, als wenn zwischen der zweiten Opferschicht und der abgeschiedenen Metallschicht noch eine Zwischenschicht aufgebracht ist.

Dazu wird bei der Anwendung eines lithographischen Verfahrens weiterhin vorgeschlagen, dass die zweite Opferschicht vorzugsweise nach der Aufbringung eines Photoresists, der unter Anwendung eines lithographischen Verfahrens zu einer der gewünschten Struktur der Metallfolie entsprechenden Ätzmaske strukturiert wird, dem Trocken-Ätzprozess unterzogen wird, wobei der Photoresist vor dem Aufbringen der Metallschicht von der zweiten Opferschicht entfernt wird. So kommt die abgeschiedene Metallschicht unmittelbar in Kontakt mit der zweiten Opferschicht.

Besonders vorteilhaft ist es dabei ferner, wenn die nasschemische Ätzung der ersten Opferschicht durch den aufgebrachten Photoresist hindurch erfolgt, der erst nach der nass-chemischen Ätzung von der ersten Opferschicht entfernt wird. Der Photoresist dient so für beide Ätzschritte als Ätzmaske.

Um eine bessere Handhabbarkeit zu erreichen wird vorgeschlagen, dass der Opferschichtverbund vor der Beschichtung mit der Metallschicht auf ein Substrat aufgebracht wird, welches insbesondere durch einen Silizium-Wafer gebildet sein kann.

Gemäß einer besonders bevorzugten Ausführungsform der Erfindung ist vorgesehen, dass die erste Opferschicht eine Dicke aufweist, die ein Vielfaches der Dicke der zweiten Opferschicht beträgt. Auf diese Weise können ausreichend große Ausnehmungen in die erste Opferschicht eingeätzt werden, so dass Kurzschlüsse der in der Netz-Struktur der Metallfolie vorgesehenen Freiräume beim Sputtern sicher vermieden werden.

Vorzugsweise wird hierzu vorgeschlagen, dass die erste Opferschicht eine Dicke von mehr als 5 µm, insbesondere eine Dicke zwischen 10 µm und 50 µm, und die zweite Opferschicht eine Dicke zwischen 0,05 µm und 1 µm, insbesondere zwischen 0,1 µm und 0,5 µm aufweist.

Vorzugsweise wird die Metallschicht mittels Sputtern, insbesondere mittels Magnetron-Sputtern, auf den strukturierten Opferschichtverbund abgeschieden.

Dabei ist es besonders günstig, wenn die Metallschicht in einer Dicke zwischen 0,1 µm und 200 µm, insbesondere zwischen 1 µm und 100 µm, und vorzugsweise zwischen 5 µm und 50 µm, aufgebracht wird.

Vorteilhaft kann es ferner sein, wenn auf die abgeschiedene Metallschicht mindestens eine Deckschicht aufgebracht wird, die aus einem von der Legierung der Metallschicht verschiedenen Material oder aus einer davon verschiedenen Legierung besteht.

Nach einer besonders bevorzugten Ausführungsform der Erfindung ist jedoch vorgesehen, dass in die Metallschicht mindestens eine Kernschicht eingebettet wird, die aus einem von der Legierung der Metallschicht verschiedenen Material oder aus einer davon verschiedenen Legierung besteht. Bei dem Kern kann es sich beispielsweise um magnetisches oder auch um ein Material handeln, welches eine bessere Röntgensichtbarkeit besitzt als Nickel-Titan-Legierungen. Durch die vollständige Einbettung der Kernschicht in die Nickel-Titan-Schicht bleibt die Biokompatibilität der Metallschicht unabhängig vom Material der Kernschicht erhalten, was in der Medizintechnik von entscheidender Bedeutung ist.

Vorzugsweise wird dabei auch die Deckschicht bzw. die Kernschicht mittels Sputtern, insbesondere mittels Magnetron-Sputtern, auf die Metallschicht abgeschieden, welche ihrerseits auf dem strukturierten Opferschichtverbund aufgebracht ist.

Günstig ist es, wenn die Dicke der Deckschicht bzw. der Kernschicht nur einen Bruchteil der Dicke der Metallschicht beträgt. Auf diese Weise werden die Formgedächtniseigenschaften bzw. das superelastische Verhalten der Nickel-Titan-Schicht nur minimal durch die zusätzliche(n) Schicht(en) beeinflusst.

Hierzu wird vorzugsweise vorgeschlagen, dass die Deckschicht bzw. die Kernschicht in einer Dicke zwischen 1 nm und 2 µm, insbesondere zwischen 0,01 µm und 0,2 µm, und vorzugsweise zwischen 0,02 µm und 0,1 µm, aufgebracht wird.

Besonders vorteilhaft ist es bei der Einbettung einer Kernschicht, wenn die Kernschicht zunächst auf eine erste Metallschicht aufgebracht wird, wobei die Kernschicht danach, insbesondere unter Aufbringung eines lithographisch zu einer Ätzmaske strukturierten Photoresists, an einigen oder vorzugsweise an allen Randbereichen einem Ätzprozess, vorzugsweise einem Trocken-Ätzprozess, unterzogen wird, und danach sowie nach der Entfernung eines gegebenenfalls aufgebrachten Photoresists eine zweite Metallschicht auf die Kernschicht und die erste Metallschicht aufgebracht wird. Die erste und die zweite Metallschicht bilden dabei zusammen die erfindungsgemäß hergestellte Nickel-Titan-Schicht. Auf diese Weise ist stets eine vollständige Umhüllung der Kernschicht durch die Nickel-Titan-Legierung und somit die Biokompatibilität gewährleistet. Bei einer einfacheren Ausführungsvariante des erfindungsgemäßen Verfahrens kann die Einbettung der Kernschicht jedoch auch ohne separate Ätzung der Kernschichtränder erfolgen.

Gemäß einer weiteren besonders bevorzugten Ausführungsform der Erfindung wird der Opferschichtverbund nach dem Sputtern von der Metallschicht entfernt und/oder aufgelöst. Auf diese Weise wird eine freitragende Metallschicht bzw. ein freitragender Film erhalten.

Besonders vorteilhaft ist es ferner, wenn die Metallschicht als dreidimensionalen Körper auf einen dreidimensionalen, insbesondere zylinderförmigen und rotierenden Opferschichtverbund abgeschieden oder nach einer ebenen Abscheidung zu einem dreidimensionalen Körper geformt und/oder zusammengefügt wird. So kann eine erfindungsgemäß hergestellte Metall-Folie insbesondere nach einer stoffschlüssigen Verbindung, beispielsweise nach Verschweißung oder Verklebung zu einem Rohr als Stent eingesetzt werden.

Zur Bildung von Strukturen mit rundem Querschnitt, insbesondere zur Herstellung von durch Sputtern hergestellten, freitragenden Stents kann der Opferschichtverbund auf einem zylindrischen Substrat aufgebracht werden. Dabei kann eine radial außen angeordnete Opferschicht zur Bildung einer Struktur entsprechend der Struktur der herzustellenden Metallschicht bereichsweise abgetragen, insbesondere geätzt werden. Die radial außen angeordnete Opferschicht bietet die Voraussetzung dafür, dass eine Trägerstruktur hergestellt werden kann, die präzise gefertigte Kanten bzw. Konturen aufweist. Zur Bildung der Trägerstruktur kann auf die radial außen angeordnete Opferschicht eine photolithografische Schicht aufgebracht werden, die nach Belichtung und Entwicklung die Struktur entsprechend der Struktur der herzustellenden Metallschicht bestimmt. Die Verwendung eines photolithografischen Verfahrens ermöglicht die Bildung einer Ätzmaske auf der radial außen angeordneten Opferschicht, die den Anforderungen an die Ausbildung einer präzisen Trägerstruktur genügt. Zur Bildung der Struktur entsprechend der Struktur der herzustellenden Metallschicht kann ein Trocken-Ätzverfahren, insbesondere ein Ionenstrahlätzen (ion beam etching) und/oder ein reaktives lonenätzen (reactive ion etching) eingesetzt werden. Derartige Trocken-Ätzverfahren erlauben vorteilhafterweise die Bildung geometrisch präziser Kanten, die in nachfolgenden Verfahrensschritten bei der Beschichtung der Trägerschicht mit Metall in der Struktur der Metallschicht abgebildet werden.

Eine radial innen angeordnete Opferschicht kann zur Bildung einer Struktur entsprechend der Struktur der herzustellenden Metallschicht bereichsweise abgetragen, insbesondere geätzt werden. Die radial innen angeordnete Opferschicht bietet die Voraussetzung dafür, dass ausreichend viel Material aus dem Opferschichtverbund abgetragen werden kann, um die Bildung und Ablösung der freitragenden gesputterten Metallschicht vom Substrat zu ermöglichen. Die Aufteilung des Opferschichtverbundes in eine radial außen und eine radial innen angeordnete Opferschicht ermöglicht eine Feinstrukturierung zuerst in der radial außen angeordneten Opferschicht, die als eigentliche Trägerschicht für die Metallbeschichtung fungiert, wobei die erforderliche Abtragstiefe durch Ätzen bzw. allgemein durch Abtragen der radial innen angeordneten Opferschicht erreicht wird.

Es erfolgt daher eine Funktionsauftrennung durch den Opferschichtverbund, wobei die radial außen angeordnete Opferschicht für die Feinstrukturierung und die radial innen angeordnete Opferschicht für die ausreichende Abtragstiefe verantwortlich sind.

Zur Bildung der Struktur entsprechend der Struktur der herzustellenden Metallschicht kann ein nasschemisches Ätzverfahren eingesetzt werden, das üblicherweise ausreichend hohe Abtragsraten ermöglicht, die die gewünschte Abtragstiefe der radial innen angeordneten Opferschicht in angemessener Zeit ermöglicht.

Die photolithografische Schicht kann nach der Bearbeitung der Opferschichten entfernt werden, so dass die Metallschicht direkt auf die radial außen angeordnete Opferschicht aufgebracht, insbesondere aufgesputtert werden kann.

Die Metallschicht, insbesondere eine Nickel-Titan-Legierung, wird vorteilhafterweise zumindest auf die radial außen angeordnete Opferschicht aufgebracht. Dabei ist nicht ausgeschlossen, dass die Metallschicht auch auf das Substrat aufgrund der Strukturierung der radial außen angeordneten Opferschicht aufgebracht wird, was insbesondere bei der Beschichtung mittels Sputtern der Fall ist. Zur Bildung einer freitragenden strukturierten Metallschicht wird vorteilhafterweise der Opferschichtverbund entfernt.

Weitere Vorteile und Merkmale der Erfindung ergeben sich aus der nachfolgenden Beschreibung und den in den Zeichnungen dargestellten Ausführungsbeispielen. Es zeigen:
- Figuren 1 bis 7: eine schematische Darstellung einer ersten Ausführungsform des erfindungsgemäßen Herstellungsverfahrens;
- Figuren 8 bis 11: eine schematische Darstellung einer ersten Variante des in den Figuren 1 bis 7 gezeigten Herstellungsverfahrens;
- Figuren 12 bis 17: eine weitere Variante des in den Figuren 8 bis 10 dargestellten Herstellungsverfahrens; und
- Figuren 18 bis 28: eine schematische Darstellung einer zweiten Ausführungsform des erfindungsgemäßen Herstellungsverfahren.

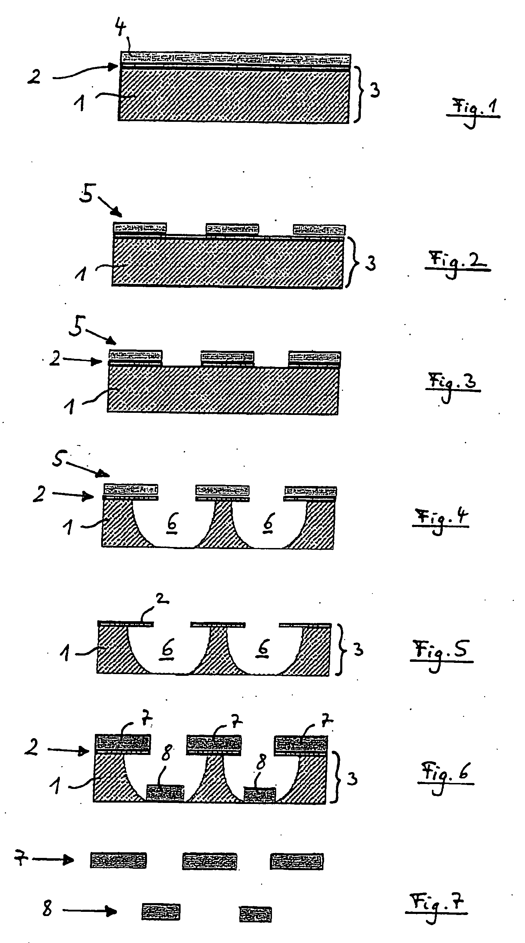

Nachfolgend wird zunächst das in den Figuren 1 bis 7 schematisch dargestellte grundlegende Verfahren zur Herstellung eines Dünnschicht-Nickel-Titan-Netzes beschrieben. Zunächst wird auf ein hier nicht dargestelltes Substrat eine erste Opferschicht 1 in einer Dicke von über 10 µm abgeschieden. Unmittelbar darauf wird eine wesentlich dünnere zweite Opferschicht 2 aus einem Material, welches sich chemisch selektiv zur Opferschicht 1 entfernen lässt, mit einer Dicke von ca. 1 µm abgeschieden. Die beiden Opferschichten 1 und 2, die beispielsweise aus Gold, Kupfer oder Chrom bestehen können, bilden zusammen einen Opferschichtverbund 3, auf den nun die gewünschte Struktur mittels eines lithographischen Verfahrens aufgebracht wird. Dazu wird auf die obere Opferschicht 2 zunächst eine Photolackschicht 4 als Photoresist aufgebracht (Fig. 1).

Diese Photolackschicht 4 wird sodann unter Anwendung eines lithographischen Verfahrens mit einer geeigneten Lithographiemaske und einer geeigneten Belichtungsquelle zu einer der gewünschten Struktur der abzuscheidenden Metallfolie entsprechenden Ätzmaske 5 strukturiert (Fig. 2).

Anschließend wird unter Verwendung der Ätzmaske 5 in einem ersten Ätzschritt, der als Trocken-Ätzprozess ausgeführt wird, die oben liegende zweite Opferschicht 2 strukturiert (Fig. 3).

Danach wird die untere erste Opferschicht 1 ebenfalls noch unter Verwendung der Ätzmaske 5 in einem zweiten Ätzschritt nass-chemisch geätzt und unter Ausbildung von Freiräumen 6 bis zum Substrat entfernt. Hierbei erfolgt eine beabsichtige Unterätzung, die als Hinterschneidung unter der oberen Opferschicht 2 im weiteren Prozessverlauf ausgenutzt wird (Fig. 4).

Nach Abschluss der beiden Ätz-Prozesse wird die Ätzmaske 5 bzw. die entsprechenden Reste des Photolacks 4 beispielsweise unter Anwendung eines Aceton-Bades entfernt, so dass der fertig vorstrukturierte Opferschichtverbund 3 mit den gewünschten Freiräumen 6 in der unteren Opferschicht 1 verbleibt (Fig. 5).

Danach erfolgt der hier mittels Kathodenzerstäuben durchgeführte Sputtervorgang, bei dem die Nickel-Titan-Legierung in einer Dicke von ca. 3 µm direkt als strukturierte Metallschicht 7 bzw. als strukturierte Metallfolie auf die verbliebenen Reste der oberen Opferschicht 2 sowie als ein der Struktur der Metallschicht 7 entsprechendes Negativabbild 8 in die Freiräume 6 in der unteren Opferschicht 1 abgeschieden wird (Fig. 6). Eine Mikrostrukturierung kann dabei zusätzlich auch noch durch eine Variation von Prozessparametern, beispielsweise des Sputtergasdrucks während des Sputtervorgangs erreicht werden. Der Nickelgehalt in der Nickel-Titan-Legierung liegt hier bei 51 Atom-%.

Abschließend werden die beiden Opferschichten 1 und 2 vorzugsweise in einem chemischen Bad aufgelöst, so dass die strukturierte Nickel-Titan-Legierungsschicht 7 als freistehende Netzstruktur erhalten wird (Fig. 7). Diese erfingdungsgemäß hergestellte strukturierte Metallfolie 7 kann nach geeigneter Formgebung vorteilhafterweise im medizinischen Bereich als Implantat, insbesondere als Stent eingesetzt werden.

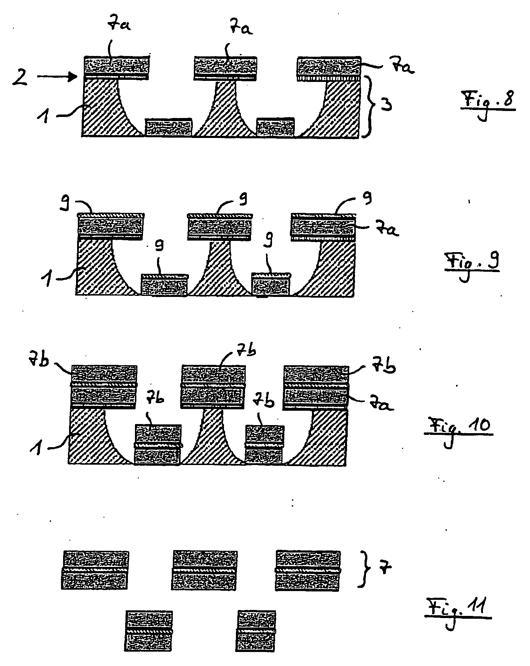

Bei der in den Figuren 8 bis 11 dargestellten Variante wird in die strukturierte Metallfolie 7 eine Kernschicht 9 aus einer anderen Metalllegierung eingebettet, um eine bessere Röntgensichtbarkeit eines daraus gebildeten Stents zu erzielen. Dazu wird auf eine nach dem Verfahren der vorangehend beschriebenen Art hergestellte und zuerst abgeschiedene erste Metallfolie 7a (Fig. 8) eine Kernschicht 9 mit einer Dicke von ca. 1 µm ebenfalls durch Sputtern aufgebracht (Fig. 9).

Danach wird eine zweite Metallschicht 7b auf die Kernschicht 9 aufgebracht (Fig. 10). Nach dem abschließenden Entfernen der beiden Opferschichten 1, 2 bilden die erste und die zweite Metallschicht 7a, 7b zusammen mit der dazwischen liegenden Kernschicht 9 eine nach dem erfindungsgemäßen Verfahren hergestellte Nickel-Titan-Metallschicht 7 (Fig. 11).

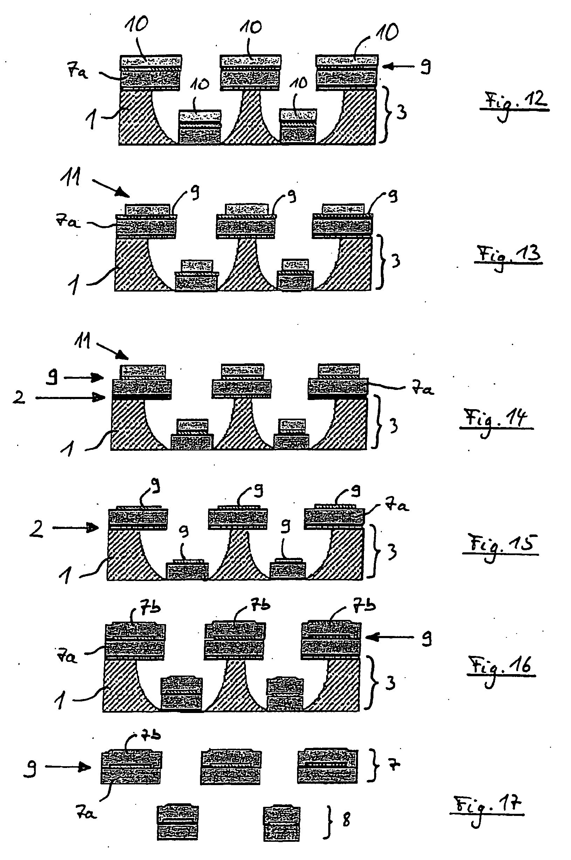

Um für eine verbesserte Biokompatibilität eine vollständige Einbettung der Kernschicht 9 in die Nickel-Titan-Schicht 7 zu erhalten, wird die in den Figuren 12 bis 17 dargestellte Abwandlung vorgeschlagen. Ausgehend von dem in Fig. 9 dargestellten Zwischenschritt wird auf die Kernschicht 9 zunächst eine Photolackschicht 10 aufgebracht (Fig. 12), die als Photoresist ebenfalls durch Belichten und Entwickeln umfassende lithographische Methoden zu einer Ätzmaske 11 strukturiert wird (Fig. 13).

Danach wird die Kernschicht 9 unter Verwendung der zuvor erzeugten Ätzmaske 11 in einem Trocken-Ätzprozess, der hier beispielsweise durch reaktives lonenätzen (RIE) ausgeführt wird, an ihren Randbereichen geätzt (Fig. 14). Dabei werden alle Ränder der Kernschicht 9 in einem Bereich weggeätzt, der etwas größer ist als die Dicke der Kernschicht 9.

Danach wird die Ätzmaske 11 vorzugsweise unter Anwendung eines Aceton-Bades entfernt (Fig. 15) und anschließend wird die zweite Metallschicht 7b auf die Kernschicht 9 sowie auf die nunmehr freiliegenden Randbereiche der ersten Metallschicht 7a aufgesputtert (Fig. 16). Deutlich ist dabei zu erkennen, dass die Kernschicht 9 auch an ihren Rändern vollständig von den beiden Metallschichten 7a, 7b umgeben ist, die nach der abschließenden Entfernung der beiden Opferschichten 1, 2 wiederum zusammen mit der dazwischen liegenden gesputterten Kernschicht 9 die nach dem erfindungsgemäßen Verfahren hergestellte Nickel-Titan-Metallschicht 7 bilden (Fig. 17).

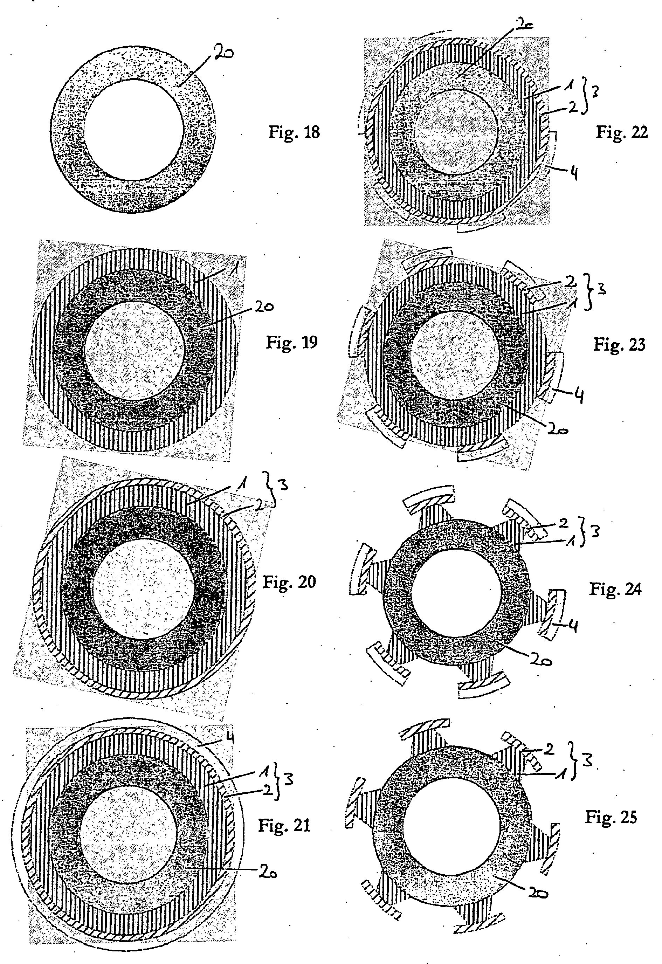

In den Figuren 18 bis 28 ist in schematischer Weise ein zweites Ausführungsbeispiel der Erfindung dargestellt, das insbesondere zur Herstellung von freitragenden Metallschichten 7 mit dreidimensionalen, d.h. nicht planaren Strukturen vorgesehen ist. Das zweite Ausführungsbeispiel ist insbesondere zur Herstellung von freitragenden Stents geeignet, die durch Sputtern oder andere Beschichtungstechniken hergestellt sind. Im Unterschied zu herkömmlichen Stents, die beispielsweise eine gesputterte Beschichtung aufweisen, besitzen die mit dem nachstehend beschriebenen Verfahren herstellbaren Stents keine Stützstruktur, sondern sind vollständig aus einer durch Sputtern gebildeten Gitterstegstruktur aufgebaut.

Dabei wird das vorstehend beschriebene erfindungsgemäße Prinzip genutzt, bei dem zur Bildung einer strukturierten Trägerschicht für die nachfolgende Beschichtung mit dem Stentmaterial ein Opferschichtverbund 3 verwendet wird. Der Opferschichtverbund 3 umfasst mindestens zwei Opferschichten 1, 2, die einer Feinstrukturierung und einer Grobstrukturierung unterzogen werden. Bei der Feinstrukturierung wird eine Struktur gebildet, die der Struktur der herzustellenden Metallschicht 7 entspricht und sehr präzise hergestellt ist. Dafür genügt eine geringe Abtragstiefe, so dass die für die Feinstrukturierung vorgesehene Opferschicht 2 entsprechend dünn sein kann. Für die Bildung einer ausreichenden Wandstärke des Stents bzw. allgemein der strukturierten Metallschicht 7 ist eine Abtragstiefe des Opferschichtverbundes 3 erforderlich, die größer ist, als die für die Feinstrukturierung verwendete Abtragstiefe. Um die dafür erforderliche Abtragstiefe zu erreichen, wird ein anderes Abtragsverfahren auf die Opferschicht 1 angewandt, das entsprechend hohe Abtragsraten ermöglicht.

Der Kern der Erfindung besteht also darin, ein Verfahren zur Herstellung von strukturierten Metallschichten 7, insbesondere zur Herstellung von Stents anzugeben, bei dem ein Opferschichtverbund 3 mit mindestens zwei Opferschichten 1, 2 verwendet wird, die mit unterschiedlichen Abtragsverfahren strukturiert werden, wobei eine Opferschicht 1, 2 durch ein Abtragsverfahren mit geringer Abtragsrate feinstrukturiert und die andere Opferschicht 2, 1 durch ein Abtragsverfahren mit hoher Abtragsrate grob strukturiert wird.

Vorzugsweise wird die Metallschicht 7 als dreidimensionaler Körper auf einen dreidimensionalen, insbesondere zylinderförmigen, Opferschichtverbund 3 abgeschieden oder zu einem dreidimensionalen Körper geformt und/oder zusammengefügt.

Zur Herstellung von freitragenden gesputterten Stents wird ein Substrat 20 mit kreisförmigem Querschnitt verwendet, beispielsweise ein hohlzylindrisches oder vollzylindrisches Substrat 20. Das Substrat 20 kann aus Glas oder Metall, beispielsweise aus Kupfer hergestellt sein (Fig. 18).

Auf das Substrat 20 wird der Opferschichtverbund 3 aufgebracht bzw. abgeschieden. Dazu wird zunächst eine Opferschicht 1 direkt auf dem Substrat 20 abgeschieden, die im Opferschichtverbund 3 radial innen angeordnet ist und eine erste Opferschicht 1 bildet (Fig. 19). Auf der ersten Opferschicht 1 wird ein zweite Opferschicht 2 abgeschieden, die im Opferschichtverbund 3 radial außen angeordnet ist. Das bedeutet, dass die erste Opferschicht 1 zwischen dem Substrat 20 und der außen angeordneten Opferschicht 2 angeordnet ist (Fig. 20).

Dabei ist nicht ausgeschlossen, dass weitere Opferschichten vorgesehen sind.

In Fig. 20 ist deutlich zu erkennen, dass die radial außen angeordnete zweite Opferschicht 2 eine kleinere Wandstärke als die radial innen angeordnete erste Opferschicht 1 aufweist. Die Wandstärke der radial außen angeordneten Opferschicht 2 entspricht etwa der Wandstärke des herzustellenden freitragenden Stents. Die Wandstärke der radial innen ersten Opferschicht 1 entspricht mindestens der Wandstärke der radial außen angeordneten zweiten Opferschicht 2. Insbesondere liegt das Verhältnis der Wandstärke der radial außen angeordneten zweiten Opferschicht 2 zur Wandstärke der radial innen angeordneten ersten Opferschicht 1 in einem Bereich von 1:1 bis 1:5, insbesondere 1:2 bis 1:4.

Auf der radial außen angeordneten Opferschicht 2 wird eine photolithografische Schicht 4 bzw. ein Photolack oder Photoresist aufgebracht (Fig. 21). Zur Bildung einer Ätzmaske 5 wird die fotolithografische Schicht 4 belichtet und entwickelt, wobei die dadurch erzeugte Ätzmaske 5 der Struktur insbesondere der Gitterstruktur der herzustellenden Metallschicht 7 bzw. des herzustellenden freitragenden Stents entspricht (Fig. 22).

Nach der Bildung der Ätzmaske (Fig. 22) wird die radial außen angeordnete zweite Opferschicht 2 feinstrukturiert, wobei eine der Ätzmaske entsprechende Struktur der zweiten Opferschicht 2 erzeugt wird. Dazu wird ein Abtragsverfahren, insbesondere ein Ätzverfahren verwendet, das eine entsprechende Feinstrukturierung ermöglicht, insbesondere die Bildung von geometrisch präzisen Kanten, ohne dass es dabei zu nennenswerten Hinterschneidungen kommt. Das für die Feinstrukturierung verwendete Ätzverfahren kann eine vergleichsweise geringe Abtragsrate aufweisen, ohne dass dabei der Herstellungsprozess verzögert wird, da die Grobstrukturierung bzw. die Erzeugung einer ausreichenden Abtragstiefe in einem weiteren Verfahrensschritt durch ein anderes Abtragsverfahren erzielt wird (Fig. 24).

Für die Feinstrukturierung der radial außen angeordneten Opferschicht 2 wird ein Trocken-Ätzverfahren verwendet, insbesondere Ionenstrahlätzen bzw. ion beam etching (IBE) und/oder reaktives lonenätzen bzw. reaktive ion etching (RIE). Andere Abtragsverfahren, die eine präzise Feinstrukturierung ermöglichen, können ebenfalls eingesetzt werden.

Die erforderliche Ätz- bzw. Abtragstiefe wird durch die Anwendung eines anderen Abtragsverfahrens bzw. Ätzverfahrens auf die innen angeordnete dickere erste Opferschicht 1 erreicht (Fig. 24). Dabei wird ein Verfahren eingesetzt, das eine entsprechend hohe Abtrags- bzw. Ätzrate aufweist, beispielsweise ein nasschemisches Ätzverfahren. Die dabei auftretenden Hinterschneidungen bzw. die Bildung der groben Kantenstruktur im Bereich der radial innen angeordneten ersten Opferschicht 1 ist unschädlich, da die für die Abbildung der Gitterstruktur der Metallschicht 7 bzw. des herzustellenden Stents verantwortliche radial außen angeordnete zweite Opferschicht 2 feinstrukturiert ist und durch die Grobstrukturierung der radial innen angeordneten Opferschicht 1 nicht beeinflusst wird. Nachdem sowohl die Feinstrukturierung (Fig. 23) als auch die Grobstrukturierung (Fig. 24) abgeschlossen sind, kann die fotolithographische Schicht 4 entfernt werden (Fig. 25), so dass die radial außen angeordnete feinstrukturierte Opferschicht 2 für die nachfolgende Metallbeschichtung freiliegt.

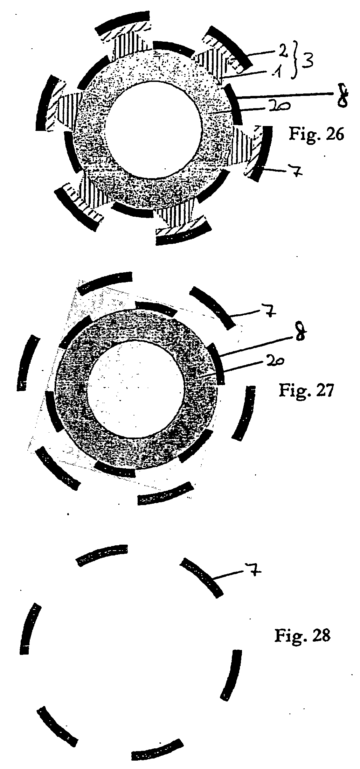

Im Anschluss daran erfolgt die Beschichtung des strukturierten Opferschichtverbundes 3 mit dem Stentmaterial. Die Beschichtung erfolgt durch ein übliches Beschichtungsverfahren, vorzugsweise durch Sputtern. Andere PVD- oder CVD-Verfahren (physical vapor deposition/chemical vapor deposition) sind für die Aufbringung des Stentmaterials auf die radial außen angeordnete zweite Opferschicht 2 möglich. Die bei der Feinstrukturierung der radial außen angeordneten Opferschicht 2 gebildeten geometrisch präzisen Kanten bzw. allgemein die geometrisch präzise Kontur der strukturierten Trägerschicht wird auf die Struktur der Metallschicht 7 abgebildet. Auf diese Weise ist es möglich, eine formgenaue Gitterstruktur des Stents durch ein Beschichtungsverfahren insbesondere durch Sputtern herzustellen (Fig. 26). Wie ferner in Fig. 26 dargestellt, wird das Stentmaterial 8 auf dem Substrat 20 in den Freiräumen zwischen den beschichteten Trägerstrukturen bzw. im beschichteten Opferschichtverbund 3 abgeschieden. Das direkt auf dem Substrat 20 abgeschiedene Stentmaterial 8 bildet somit eine Negativform des auf dem Opferschichtverbund 3 abgeschiedenen Materials 7 bzw. der Metallschicht 7. Die grobstrukturierte relativ dicke erste Opferschicht 1 ist so dimensioniert, dass ein ausreichender Abstand zwischen der auf der feinstrukturierten Opferschicht 2 abgeschiedenen Metallschicht 7 und dem auf dem Substrat 20 abgeschiedenen Stentmaterial 8 vorgesehen ist, um zu vermeiden, dass Verbindungen oder Brücken zwischen der Metallschicht 7 und dem direkt auf dem Substrat 20 abgeschiedenen Stentmaterial 8 bestehen.

Zum Ablösen des freitragenden Stents bzw. allgemein der freitragenden strukturierten Metallschicht 7 wird der Opferschichtverbund 3 entfernt, insbesondere vollständig durch Ätzen abgetragen. Dadurch kommt die strukturierte Metallschicht 7 bzw. der freitragende Stent vom Substrat 20 frei und kann einfach abgezogen werden (Fig. 28).

Das vorstehend beschriebene Verfahren eignet sich besonders zur Herstellung von freitragenden gesputterten Stents. Das Verfahren kann auch auf andere nicht planare Strukturen übertragen werden, wobei das Substrat zur Aufbringung bzw. zur Bildung des Opferschichtverbundes entsprechend angepasst wird. Das Verfahren ist daher sehr flexibel und bietet die Voraussetzung für die Herstellung unterschiedlicher geometrischer Strukturen. Alle Merkmale, die im Zusammenhang mit dem zweiten Ausführungsbeispiel offenbart sind, werden auch, soweit technisch sinnvoll, im Zusammenhang mit den anderen Ausführungsbeispielen offenbart und umgekehrt.

### Bezugszeichenliste

- 1: erste Opferschicht
- 2: zweite Opferschicht
- 3: Opferschichtverbund
- 4: Photoresist
- 5: Ätzmaske
- 6: Freiraum
- 7: Metallschicht
- 7a: erste Metallschicht
- 7b: zweite Metallschicht
- 8: Negativabbild
- 9: Kernschicht
- 10: Photolackschicht
- 11: Ätzmaske
- 20: Substrat

## Patentansprüche

1. Verfahren zur Herstellung einer strukturierten Metallschicht (7) aus einer Titan und Nickel umfassenden Legierung, umfassend die folgenden Verfahrensschritte:
- ein Opferschichtverbund (3) wird bereitgestellt, welcher eine auf eine erste Opferschicht (1) aufgebrachte zweite Opferschicht (2) umfasst,
- eine der beiden Opferschichten (1) wird zur Strukturierung einem nass-chemischen Ätzprozess derart unterzogen, dass eine Unterätzung der zweiten Opferschicht (2) auftritt,
- eine Metallschicht (7) der besagten Legierung wird mittelbar oder unmittelbar auf den strukturierten Opferschichtverbund (3) aufgebracht,
**dadurch gekennzeichnet, dass**
die von der Metallschicht (7) weiter entfernt liegende erste Opferschicht (1) dem nass-chemischen Ätzprozess unterzogen wird, und dass die der Metallschicht (7) zugewandte zweite Opferschicht (2) vor dem nass-chemischen Ätzen der ersten Opferschicht (1) einem Trocken-Ätzprozess unterzogen wird, wobei sie mit einer der gewünschten Struktur der Metallschicht (7) entsprechenden Struktur versehen wird.

2. Verfahren nach Anspruch 1,
**dadurch gekennzeichnet, dass**
die zweite Opferschicht (2) nach der Aufbringung eines Photoresists (4), der unter Anwendung eines lithographischen Verfahrens zu einer der gewünschten Struktur der Metallfolie (7) entsprechenden Ätzmaske (5) strukturiert wird, dem Trocken-Ätzprozess unterzogen wird, und dass der Photoresist (4) vor dem Aufbringen der Metallschicht (7) von der zweiten Opferschicht (2) entfernt wird.

3. Verfahren nach Anspruch 2,
**dadurch gekennzeichnet, dass**
die nass-chemische Ätzung der ersten Opferschicht (1) durch den aufgebrachten Photoresist (4) hindurch erfolgt, der erst nach der nass-chemischen Ätzung von der ersten Opferschicht (1) entfernt wird.

4. Verfahren nach einem der vorherigen Ansprüche,
**dadurch gekennzeichnet, dass**
auf die Metallschicht (7) mindestens eine Deckschicht aufgebracht wird, die aus einem von der Legierung der Metallschicht (7) verschiedenen Material oder aus einer davon verschiedenen Legierung besteht.

5. Verfahren nach einem der vorherigen Ansprüche,
**dadurch gekennzeichnet, dass**
in die Metallschicht (7) mindestens eine Kernschicht (9) eingebettet wird, die aus einem von der Legierung der Metallschicht (7) verschiedenen Material oder aus einer davon verschiedenen Legierung besteht.

6. Verfahren nach Anspruch 4 oder 5,
**dadurch gekennzeichnet, dass**
die Deckschicht und/oder die Kernschicht (9) mittels Sputtern, insbesondere mittels Magnetron-Sputtern, auf die Metallschicht (7) abgeschieden wird, welche auf dem strukturierten Opferschichtverbund (3) aufgebracht ist.

7. Verfahren nach einem der Ansprüche 4 bis 6,
**dadurch gekennzeichnet, dass**
die Dicke der Deckschicht und/oder der Kernschicht (9) einen Bruchteil der Dicke der Metallschicht (7) beträgt.

8. Verfahren nach einem der Ansprüche 5 bis 7,
**dadurch gekennzeichnet, dass**
die Kernschicht (9) auf eine erste Metallschicht (7a) aufgebracht wird, dass die Kernschicht (9) danach, insbesondere unter Aufbringung eines lithographisch zu einer Ätzmaske (11) strukturierten Photoresists (10), an zumindest einigen Randbereichen einem Ätzprozess, vorzugsweise einem Trocken-Ätzprozess, unterzogen wird, und dass danach sowie nach der Entfernung eines gegebenenfalls aufgebrachten Photoresists (10) eine zweite Metallschicht (7b) auf die Kernschicht (9) und die erste Metallschicht (7a) aufgebracht wird.

9. Verfahren nach wenigstens einem der Ansprüche 1 bis 8,
**dadurch gekennzeichnet, dass**
der Opferschichtverbund (3) auf ein zylindrisches Substrat (20) aufgebracht wird.

10. Verfahren nach Anspruch 9,
**dadurch gekennzeichnet, dass**
eine radial außen angeordnete Opferschicht (2) und/oder eine radial innen angeordnete Opferschicht (2) zur Bildung einer Struktur entsprechend einer Struktur der herzustellenden Metallschicht (7) bereichsweise abgetragen, insbesondere geätzt, wird.

11. Verfahren nach Anspruch 10,
**dadurch gekennzeichnet, dass**
auf die radial außen angeordnete Opferschicht (2) eine photolithographische Schicht (4) aufgebracht wird, die nach Belichtung und Entwicklung die Struktur entsprechend der Struktur der herzustellenden Metallschicht (7) bestimmt.

12. Verfahren nach wenigstens einem der Ansprüche 9 bis 11,
**dadurch gekennzeichnet, dass**
die Metallschicht (7), insbesondere eine Nickel-Titan-Legierung, zumindest auf die radial außen angeordnete Opferschicht (2) aufgebracht wird.

13. Verfahren nach wenigstens einem der Ansprüche 9 bis 12,
**dadurch gekennzeichnet, dass**
der Opferschichtverbund (3) zur Bildung einer freitragenden strukturierten Metallschicht (7) entfernt wird.

## Claims

1. A method for the production of a structured metal layer (7) made of an alloy containing titanium and nickel, comprising the following production steps:
- a sacrificial layer composite (3) comprising a second sacrificial layer (2) applied onto a first sacrificial layer (1) is provided,
- one of the two sacrificial layers (1) is subjected to a wet-chemical etching process for structuring purposes such that undercutting of the second sacrificial layer (2) occurs, and
- a metal layer (7) of said alloy is directly or indirectly applied onto the structured sacrificial layer composite (3),
**characterized in that**
the first sacrificial layer (1), which lies farther from the metal layer (7), is subjected to the wet-chemical etching process, and **in that** the second sacrificial layer (2) facing the metal layer (7) is prior to the wet-chemical etching of the first sacrificial layer (1) subjected to a dry-etching process, in which it is provided with a structure corresponding to the desired structure of the metal layer (7).

2. The method according to claim 1,
**characterized in that**
the second sacrificial layer (2) is subjected to the dry-etching process after the application of a photoresist (4), which is structured into an etching mask (5) corresponding to the desired structure of the metal film (7) by using a lithographic method, and **in that** the photoresist (4) is removed from the second sacrificial layer (2) prior to the application of the metal layer (7).

3. The method according to claim 2,
**characterized in that**
the wet-chemical etching of the first sacrificial layer (1) takes place through the applied photoresist (4), which is not removed from the first sacrificial layer (1) until the wet-chemical etching process is completed.

4. The method according to one of the preceding claims,
**characterized in that**
at least one cover layer, which consists of a material or of an alloy that differs from the alloy of the metal layer (7), is applied onto the metal layer (7).

5. The method according to one of the preceding claims,
**characterized in that**
at least one core layer (9), which consists of a material or of an alloy that differs from the alloy of the metal layer (7), is embedded in the metal layer (7).

6. The method according to claim 4 or 5,
**characterized in that**
the cover layer and/or the core layer (9) is deposited onto the metal layer (7), which is applied onto the structured sacrificial layer composite (3), by means of sputtering, particularly by means of magnetron sputtering.

7. The method according to one of claims 4 to 6,
**characterized in that**
the thickness of the cover layer and/or the core layer (9) amounts to a fraction of the thickness of the metal layer (7).

8. The method according to one of claims 5 to 7,
**characterized in that**
the core layer (9) is applied onto a first metal layer (7a), **in that** the core layer (9) is subsequently subjected to an etching process, preferably a dryetching process, on at least a few edge regions, particularly after applying a photoresist (10) that is lithographically structured into an etching mask (11), and **in that** a second metal layer (7b) is applied onto the core layer (9) and the first metal layer (7a) after said etching process and after the removal of a potentially applied photoresist (10).

9. The method according to at least one of claims 1 to 8,
**characterized in that**
the sacrificial layer composite (3) is applied onto a cylindrical substrate (20).

10. The method according to claim 9,
**characterized in that**
a radially outer sacrificial layer (2) and/or a radially inner sacrificial layer (2) is sectionally removed, particularly by means of etching, in order to create a structure corresponding to the structure of the metal layer (7) to be produced.

11. The method according to claim 10,
**characterized in that**
a photolithographic layer (4), which after exposure and development defines the structure corresponding to the structure of the metal layer (7) to be produced, is applied onto the radially outer sacrificial layer (2).

12. The method according to at least one of claims 9 to 11,
**characterized in that**
the metal layer (7), particularly a nickel-titanium alloy, is applied at least onto the radially outer sacrificial layer (2).

13. The method according to at least one of claims 9 to 12,
**characterized in that**
the sacrificial layer composite (3) is removed in order to create a self-supporting structured metal layer (7).

## Revendications

1. Procédé destiné à fabriquer une couche métallique (7) structurée en un alliage comprenant du titane et du nickel, comprenant les étapes de procédé suivantes :
- on prépare un stratifié de couches sacrificielles (3) lequel comprend une deuxième couche sacrificielle (2) appliquée sur une première couche sacrificielle (1),
- pour la structuration, on soumet l'une (1) des deux couches sacrificielles à un procédé chimique humide de gravure, de telle sorte qu'il apparaisse une gravure sous-jacente de la deuxième couche sacrificielle (2),
- on applique indirectement ou directement une couche métallique (7) de l'alliage cité sur le stratifié de couches sacrificielles (3) structuré,
**caractérisé en ce qu'**on soumet la première couche sacrificielle (1) la plus éloignée de la couche métallique (7) au processus de gravure chimique humide et **en ce qu'**avant la gravure chimique humide de la première couche sacrificielle (1), on soumet la deuxième couche sacrificielle (2) qui fait face à la couche métallique (7) à un processus de gravure à sec, alors qu'on la munit d'une structure correspondant à la structure souhaitée de la couche métallique (7).

2. Procédé selon la revendication 1,
**caractérisé en ce**
**qu'**après l'application d'un photorésistant (4) que l'on structure en un masque de gravure (5) correspondant à la structure souhaitée du film métallique (7) en utilisant un procédé lithographique, on soumet la deuxième couche sacrificielle (2) au processus de gravure à sec et en ce qu'on retire le photorésistant (4) de la couche métallique (7) avant l'application de la deuxième couche sacrificielle (2).

3. Procédé selon la revendication 2,
**caractérisé en ce que**
la gravure chimique humide de la première couche sacrificielle (1) s'effectue à travers le photorésistant (4) appliqué que l'on ne retire de la première couche sacrificielle (1) qu'après la gravure chimique humide.

4. Procédé selon l'une quelconque des revendications précédentes,
**caractérisé en ce**
**qu'**on applique sur la couche métallique (7) au moins une couche de couverture, qui est constituée d'une matière différente de l'alliage de la couche métallique (7) ou d'un alliage différent de celui-ci.

5. Procédé selon l'une quelconque des revendications précédentes,
**caractérisé en ce**
**qu'**on incorpore dans la couche métallique (7) au moins une couche centrale (9), qui est constituée d'une matière différente de l'alliage de la couche métallique (7) ou d'un alliage différent de celui-ci.

6. Procédé selon la revendication 4 ou la revendication 5,
**caractérisé en ce**
**qu'**on dépose la couche de couverture et/ou la couche centrale (9) par pulvérisation cathodique, notamment par pulvérisation cathodique à magnétron sur la couche métallique (7), laquelle est appliquée sur le stratifié de couches sacrificielles (3) structuré.

7. Procédé selon l'une quelconque des revendications 4 à 6,
**caractérisé en ce que**
l'épaisseur de la couche de couverture et/ou de la couche centrale (9) s'élève à une fraction de l'épaisseur de la couche métallique (7).

8. Procédé selon l'une quelconque des revendications 5 à 7,
**caractérisé en ce**
**qu'**on applique la couche centrale (9) sur une première couche métallique (7a), en ce qu'ensuite, notamment en appliquant un photorésistant (10) lithographique structuré en un masque de gravure (11), on soumet la couche centrale (9) sur au moins certaines régions marginales à un processus de gravure, de préférence à un processus de gravure à sec et en ce que par la suite, ainsi qu'après le retrait d'un photorésistant (10) appliqué le cas échéant, on applique une deuxième couche métallique (7b) sur la couche centrale (9) et la première couche métallique (7a).

9. Procédé selon au moins l'une quelconque des revendications 1 à 8,
**caractérisé en ce**
**qu'**on applique le stratifié de couches sacrificielles (3) sur un substrat cylindrique (20).

10. Procédé selon la revendication 9,
**caractérisé en ce**
**qu'**on enlève, notamment on décape par régions une couche sacrificielle (2) placée à l'extérieur en direction radiale et/ou une couche sacrificielle (2) placée à l'intérieur en direction radiale pour former une structure correspondant à une structure de la couche métallique (7) qui doit être fabriquée.

11. Procédé selon la revendication 10,
**caractérisé en ce que**
sur la couche sacrificielle (2) placée à l'extérieur en direction radiale, on applique une couche photolithographique (4), qui après exposition à la lumière et développement définit la structure correspondant à la structure de la couche métallique (7) qui doit être fabriquée.

12. Procédé selon au moins l'une quelconque des revendications 9 à 11,
**caractérisé en ce**
**qu'**on applique la couche métallique (7), notamment un alliage titane-nickel au moins sur la couche sacrificielle (2) placée à l'extérieur en direction radiale.

13. Procédé selon au moins l'une quelconque des revendications 9 à 12,
**caractérisé en ce**
**qu'**on retire le stratifié de couches sacrificielles (3) pour former un couche métallique (7) en porte-à-faux structurée.
